# EUROPÄISCHE PATENTANMELDUNG

(11) **EP 4 434 474 A1**
(43) Veröffentlichungstag der Anmeldung: **25.09.2024**
(21) Anmeldenummer: 23163809.9
(22) Anmeldetag: 23.03.2023
(51) Int. Cl.: A61B 17/00, B05B 7/06

(54) **INSTRUMENT, SYSTEM UND VERFAHREN ZUM VERSPRÜHEN VON FLÜSSIGKEITEN**

(71) Anmelder: Erbe Elektromedizin GmbH, 72072 Tübingen (DE)
(72) Erfinder: STRAUB, Frank, 72070 Tuebingen (DE); FECH, Andres, 72072 Tuebingen (DE)
(74) Vertreter: Rüger Abel Patentanwälte PartGmbB

(57) **Zusammenfassung**

Das erfindungsgemäße Instrument dient dazu, mehrere miteinander reaktive Flüssigkeiten mittels mindestens eines drallbehafteten Gasstroms zu Versprühen und außerhalb der Düsenanordnung des Instruments zu vermischen. Die Düsenanordnung des Instruments weist einen Düsenkörper mit mindestens einem Flüssigkeitsauslass, durch den eine oder mehrere Flüssigkeiten ausgebbar sind, und einer Gasaustrittsanordnung auf, durch die die mindestens eine drallbehaftete Gasströmung ausgebbar ist, wobei die Gasaustrittsanordnung den mindestens einen Flüssigkeitsauslass, vorzugsweise vollständig, umschließt.

## Beschreibung

Die Erfindung betrifft ein Instrument, vorzugsweise zur minimalinvasiven Anwendung, mit einer distal an dem Instrument angeordneten Düsenanordnung, die dazu eingerichtet ist, Flüssigkeiten mittels eines drallbehafteten Gasstroms zu versprühen sowie ein System, welches dieses Instrument aufweist. Zudem betrifft die Erfindung ein Verfahren zum Versprühen von Flüssigkeiten.

Bei chirurgischen Eingriffen im Inneren eines Patientenkörpers, insbesondere im Abdominalbereich des Patienten, kann es zu einer Ausbildung von pathologischen Verwachsungen zwischen den einzelnen Organen und/oder der Bauchwand kommen. Diese pathologischen Verwachsungen werden auch als Adhäsionen bezeichnet, welche bei den Patienten zu teilweise schwerwiegenden Komplikationen nach dem Eingriff führen können.

Zur Prävention solcher Adhäsionen werden sogenannte Adhäsionsbarrieren eingesetzt, wie beispielsweise in WO 2015/176 905 A1 beschrieben wird. Bei diesen Adhäsionsbarrieren handelt es sich um Substanzen, die auf die betroffenen Gewebebereiche aufgetragen werden können, um ein Anwachsen von anderen Gewebeschichten an dieser zu verhindern. Die Adhäsionsbarrieren werden vorteilhafterweise durch Aufsprühen aufgetragen. Vorzugsweise werden diese Adhäsionsbarrieren aus zwei Komponenten gebildet, die erst unmittelbar vor oder während des Auftragens miteinander vermischt werden.

Aus dem Stand der Technik sind verschiedene Systeme zum Versprühen mehrerer Komponenten zur Adhäsionsprävention bekannt, welche insbesondere auch im laparoskopischen Bereich Anwendung finden.

In der WO 0 009 199 A1 wird eine Vorrichtung beschrieben, die zum Versprühen einer Zweikomponentenadhäsionsbarriere ausgebildet ist. Die Vorrichtung weist separate Sprühdüsen für jede der zwei Komponenten auf. Die Komponenten werden über einen Gasstrom ausgesprüht, wobei der Gasstrom durch Umfangsspalten, die die Düsen umgeben, austritt.

Die EP 3 145 417 A1 beschreibt eine zweite Vorrichtung zum Versprühen von mindestens zwei Komponenten unter Verwendung eines Druckgases im Körperinneren eines Patienten. Die Vorrichtung weist einen Spraykopf auf, in welchem die Komponenten voneinander getrennt austreten und durch einen ebenfalls am Spraykopf austretenden Gasstrom vermischt und versprüht werden.

Zweite Sprühvorrichtungen für mehrere Komponenten werden in EP 0 951 311 B1, EP 0 302 411 A1, EP 2 739 401 A2, US 2006/189 944 A1 und EP 2 695 626 A1 beschrieben.

In der US 5,368,563 A wird außerdem eine außenmischende Düse für einen physiologischen Klebestoff beschrieben. Bei dieser Düse werden die flüssigen Komponenten innerhalb der Düse verwirbelt, so dass sich die flüssigen Komponenten beim Versprühen in dem entstehenden Sprühkegel außerhalb der Düse vermischen.

Auch in der EP 2 907 582 B1 wird eine außenmischende Düse beschrieben, bei der mindestens zwei Fluide mit unterschiedlichen Volumenströmen und/oder unterschiedlicher Viskosität versprüht werden.

Zudem beschreibt die JP 2011-194304 A eine Vorrichtung zum Versprühen von zwei einen Klebstoff bildenden Komponenten, bei welcher ein rotierender Gasstrom zum Versprühen der Komponenten verwendet wird. Die Auslassöffnung für den Gasstrom befindet sich dabei mittig zwischen den beiden separaten Auslassöffnungen für die Komponenten.

Davon ausgehend ist es Aufgabe der Erfindung, ein Instrument und ein Verfahren zum Versprühen von flüssigen Komponenten bereitzustellen, welche außerhalb des Instruments vermischt werden, bei welchem insbesondere eine verbesserte Durchmischung der flüssigen Komponenten erreicht werden kann.

Diese Aufgabe wird mit dem Instrument nach Anspruch 1, dem Verfahren nach Anspruch 14 sowie dem System nach Anspruch 15 gelöst:

Das erfindungsgemäße Instrument weist eine distal am Instrument angeordnete Düsenanordnung auf, die dazu eingerichtet ist, Flüssigkeiten mittels eines drallbehafteten Gasstroms zu versprühen. Die Düsenanordnung weist einen Düsenkörper mit einem Flüssigkeitsauslass und einer Gasaustrittsanordnung auf. Das Instrument ist dazu eingerichtet, einen Flüssigkeitsstrom durch den Flüssigkeitsauslass und den drallbehafteten Gasstrom durch die Gasaustrittsanordnung auszugeben. Die Gasaustrittsanordnung ist derart angeordnet, dass diese den Flüssigkeitsauslass, vorzugsweise vollständig, umschließt, wodurch der drallbehaftete Gasstrom einen möglichst großen Überlappungsbereich mit dem Flüssigkeitsstrom aufweist.

Der Gasstrom breitet sich von der Gasaustrittsanordnung in eine Ausbreitungsrichtung mit einem durch die Gasaustrittsanordnung festgelegten Sprühwinkel aus. Der Gasstrom ist zusätzlich zu der in Ausbreitungsrichtung zeigenden Strömungskomponente mit einer Wirbelströmung (nachfolgend auch als Drall bezeichnet) beaufschlagt. Wenn der Gasstrom die Flüssigkeitströpfchen des Flüssigkeitsstroms in dem Überlappungsbereich erfasst, werden diese von dem Gasstrom mitgerissen und vermischt. Aufgrund der dem Gasstrom aufgeprägten Wirbelströmung (dem Drall) wirken zusätzliche Scherkräfte auf die Flüssigkeitströpfchen des Flüssigkeitsstromes, wodurch die Flüssigkeitströpfchen im Flug zu feineren Flüssigkeitströpfchen zerrissen werden. Hierdurch kann ein Tröpfchenspektrum mit kleineren Tröpfchengrößen erreicht werden. Durch den drallbehafteten Gasstrom kann so eine verbesserte Durchmischung der Flüssigkeiten außerhalb der Düsenanordnung erreicht werden.

Eine Besonderheit des erfindungsgemäßen Instruments liegt darin, dass durch die Düsenanordnung ein drallbehaftete Gasstrom erzeugt wird, der zum Mischen und Versprühen der Flüssigkeiten außerhalb der Düsenanordnung verwendet wird, wobei die an der Düsenanordnung austretenden Flüssigkeiten von dem drallbehafteten Gasstrom, vorzugsweise vollumfänglich, um- und/oder durchströmt werden. Der auf den Gasstrom aufgeprägte Drall kann zu einem größeren Sprühwinkel des Gas-/Flüssigkeitsgemisches führen, wodurch das Auftragen und Verteilen des Gas-/Flüssigkeitsgemisches, insbesondere bei großen Flächen, erleichtert und beschleunigt werden kann. Durch das Beaufschlagen des Gasstroms mit einem Drall wird dem Gasstrom eine kontrollierte, zusätzliche Turbulenz aufgeprägt. Die Flüssigkeitströpfchen des von den drallbehafteten Gasstrom umströmten ersten Flüssigkeitsstroms werden mitgerissen und entlang einer Trajektorie von innen nach außen und/oder von außen nach innen getragen, wobei die Tröpfchen hierdurch aufgrund der auf sie wirkenden Scherkräfte zerstäubt werden. Der dem Gasstrom aufgeprägte Drall sorgt außerdem dafür, dass die axiale Austrittsgeschwindigkeit des Gas-/Flüssigkeitsgemisches bei einer kleinen Tröpfchengröße reduziert wird, wodurch ein gleichmäßiges Auftragen der Flüssigkeit auf das Gewebe erreicht werden kann.

Die zu versprühende Flüssigkeit ist vorzugsweise aus mehreren miteinander reaktiven Komponenten zusammengesetzt. Bei der zu versprühenden Flüssigkeit kann es sich beispielsweise um ein Gel handeln, welches im Ausgangszustand in mindestens zwei Komponenten vorliegt. Es wird bevorzugt, dass es sich bei dem Gel um ein Hydrogel handelt. Bei einem Hydrogel handelt es sich um ein wasserenthaltendes und gleichzeitig wasserunlösliches Polymer.

Es wird bevorzugt, dass das Hydrogel auf Polyurethan basiert. Die Komponenten der Flüssigkeit können z.B. ein Präpolymer und ein zur Vernetzung erforderlicher Reaktionspartner sein. Diese Komponenten reagieren bei Durchmischung miteinander, wobei die Reaktionsgeschwindigkeit, mit der diese Komponenten miteinander reagieren, vergleichsweise hoch ist. Die Komponenten können beispielsweise innerhalb von 60 s oder weniger, 50 s oder weniger, sowie 40 s oder weniger bei Zimmer- oder Körpertemperatur (vollständig) miteinander reagiert haben. Die Komponenten werden daher vorzugsweise außerhalb der Düsenanordnung vermischt, um ein Verstopfen der Düsenanordnung nach dem Gebrauch zu vermeiden.

In einem ersten Ausführungsbeispiel weist die Düsenanordnung den ersten Flüssigkeitsauslass sowie einen zweiten Flüssigkeitsauslass auf, durch welchen ein zweiter Flüssigkeitsstrom austragbar ist, wobei der zweite Flüssigkeitsauslass von dem ersten Flüssigkeitsauslass beabstandet angeordnet ist. Der zweite Flüssigkeitsauslass ist vorzugsweise ein separater Flüssigkeitsauslass. Dem ersten Flüssigkeitsauslass ist vorzugsweise eine erste Flüssigkeitskapillare, durch welche ein erster Zuführstrom der ersten Flüssigkeit zur Düsenanordnung geführt wird, und dem zweiten Flüssigkeitsauslass ist vorzugsweise eine zweite Flüssigkeitskapillare zugeordnet, durch welche ein zweiter Zuführstrom der zweiten Flüssigkeit zur Düsenanordnung geführt wird. Die beiden zu vermischenden Flüssigkeiten können so separat von einem Versorgungsgerät zu der Düsenanordnung des Instruments transportiert werden und dort außerhalb der Düsenanordnung durch den drallbehafteten Gasstrom vermischt und versprüht werden. Durch das Vermischen der beiden Flüssigkeiten außerhalb der Düsenanordnung kann ein Verstopfen der Düsenanordnung vermieden werden. Der Abstand zwischen dem ersten und dem zweiten Flüssigkeitsauslass kann zwischen 0,8 und 1,5 mm liegen, vorzugsweise beträgt er 1,4 mm, wodurch eine gute Vermischung der Flüssigkeiten erreicht werden und gleichzeitig ein Verstopfen der Düsenanordnung verhindert werden kann.

Vorzugsweise weist die Gasaustrittsanordnung eine erste Gasaustrittsöffnung auf, welche konzentrisch zu dem ersten Flüssigkeitsauslass angeordnet ist. Bei der Gasaustrittsöffnung kann es sich beispielsweise um eine Ringspaltöffnung handeln. Die Ringspaltöffnung kann eine Breite von 0,08 bis 0,15 mm, vorzugsweise eine Breite von 0,1 aufweisen. Die Gasaustrittsöffnung kann jedoch auch in mehrere Ringspaltsegmente aufgeteilt sein. Alternativ kann die Gasaustrittsanordnung mehrere Gasaustrittsöffnungen aufweisen, die auf einem konzentrisch zum ersten Flüssigkeitsauslass definierten Kreis angeordnet sind.

Insbesondere weist die Gasaustrittsanordnung eine zweite Gasaustrittsöffnung auf, welche konzentrisch zu dem zweiten Flüssigkeitsauslass angeordnet ist, wobei die erste Gasaustrittsöffnung und die zweite Gasaustrittsöffnung jeweils dazu eingerichtet sind, einen drallbehafteten Gasstrom auszugeben. Der erste Flüssigkeitsauslass und der zweite Flüssigkeitsauslass sind so jeweils von einer Gasaustrittsöffnung umschlossen. Der drallbehaftete Gasstrom erfasst jeweils die aus den Flüssigkeitsauslässen austretenden Flüssigkeiten und versprüht diese, wobei sich die beiden resultierenden Sprühkegel außerhalb der Düsenanordnung überlappen. In dem Überlappungsbereich kommt es zu einer verbesserten Durchmischung der beiden Flüssigkeitsströme.

Es wird bevorzugt, dass die durch die erste Gasaustrittsöffnung und die zweite Gasaustrittsöffnung austretenden drallbehafteten Gasströme mit einem gleichsinnig rotierenden oder einem gegeneinander rotierenden Drall beaufschlagt sind.

In einer zweiten Ausführungsform sind der erste Flüssigkeitsauslass und der zweite Flüssigkeitsauslass in einem Strömungskörper voneinander beabstandet angeordnet, welcher eine Mittelachse M definiert.

Die Gasaustrittsanordnung weist insbesondere eine den Strömungskörper umschließende Gasaustrittsöffnung auf, die vorzugsweise konzentrisch zu der Mittelachse M des Strömungskörpers angeordnet ist. Der Strömungskörper weist vorzugsweise eine konvex gestaltete Außenkontur auf, wodurch der Gasstrom der Außenkontur des Strömungskörpers s folgt und ein Mitreißen der beiden Flüssigkeiten verbessert werden kann. Die Oberfläche des Strömungskörpers s ist vorzugsweise mit einer hydrophoben Beschichtung ausgestaltet. Hierdurch kann ein Anhaften von Tropfen am distalen Ende des Instruments verhindert werden, durch das es ansonsten zu einer Ansammlung von ausgehärteten (miteinander reagierenden) Flüssigkeitstropfen kommen kann, die wiederum die Gasaustrittsöffnung und/oder die Flüssigkeitsauslässe verstopfen können.

In der ersten und/oder der zweiten Ausführungsform umhüllt den drallbehafteten Gasstrom bzw. umhüllen die drallbehafteten Gasströme den ersten Flüssigkeitsstrom und den zweiten Flüssigkeitsstrom vorzugsweise vollständig, so dass sich die Flüssigkeitsströme und die Gasströme vor der Düsenanordnung möglichst großflächig überschneiden. Hierdurch kann eine möglichst große Mischzone für die Flüssigkeiten erreicht werden.

Vorzugsweise mündet mindestens ein Gaszuführkanal in den Düsenkörper, wobei die Gasaustrittsanordnung über den Gaszuführkanal mit einem Gaszuführstrom versorgbar ist. In dem Düsenkörper ist insbesondere mindestens ein Drallerzeugungsmittel untergebracht, das dazu eingerichtet ist, den Gaszuführstrom mit einem vorbestimmten Drall zu beaufschlagen. Das Drallerzeugungsmittel kann beispielsweise als ein Drallkörper ausgebildet sein, der derart angeordnet ist, dass dem Gaszuführstrom der vorbestimmte Drall aufgeprägt wird. Das Drallerzeugungsmittel kann außerdem als ein Verbindungskanal ausgebildet sein, durch den der Gaszuführstrom derart in die Gasaustrittsöffnung eingeströmt wird, dass ihm der vorbestimmte Drall aufgeprägt wird. Der Drallwinkel, der den Winkel des Dralls zur Rotationsachse des Dralls beschreibt, kann hierbei insbesondere größer als 15°, vorzugsweise größer als 20° und besonders bevorzugt größer als 25° betragen.

Insbesondere sind der erste Flüssigkeitsauslass und/oder der zweite Flüssigkeitsauslass distal von dem Instrument vorstehend angeordnet, wodurch bei einer Unterbrechung des Sprühvorgangs ein Anhaften von Flüssigkeitströpfchen an dem distalen Instrumentenende erschwert wird. Die Gasströme überströmen die Flüssigkeitsauslässe, so dass an den Flüssigkeitsauslässen anhaftende Flüssigkeitströpfchen, auch bei einem annähernd gleichzeitigem Unterbrechen des Flüssigkeitsstroms und des Gasstroms, von dem bereits ausgeströmten Gasstrom mitgerissen werden.

Vorzugsweise weist das Instrument ferner einen länglichen Schaft auf, in welchem der Gaszuführkanal und die Flüssigkeitskapillare für jeden Flüssigkeitsauslass angeordnet sind. Das Instrument kann außerdem eine proximal angeordnete Schnittstelleneinrichtung aufweisen, die mit einem Versorgungsgerät verbindbar ist. Das Versorgungsgerät kann dazu eingerichtet sein, das Instrument mit den Flüssigkeiten sowie mit dem Gaszuführstrom zu versorgen.

Vorzugsweise weist das Instrument außerdem eine Steuereinheit auf, die dazu eingerichtet ist, den ersten Flüssigkeitsstrom sowie, falls vorhanden, den zweiten Flüssigkeitsstrom bei einem Beenden des Sprühvorgangs vor dem Gasstrom und den, falls vorhanden, zweiten Gasstrom, zu unterbrechen. Die Steuereinheit kann außerdem dazu eingerichtet sein, den ersten Gasstrom vor dem ersten Flüssigkeitsstrom bei einem Beginnen des Sprühvorgangs einzuleiten und, falls vorhanden, auch den zweiten Gasstrom vor dem zweiten Flüssigkeitsstrom einzuleiten. Beide Maßnahmen können dazu beitragen, ein Ansammeln von Flüssigkeitstropfen am distalen Ende des Instruments zu vermeiden.

Das erfindungsgemäße Verfahren zum Versprühen von Flüssigkeiten mittels eines Instruments, vorzugsweise der obigen Art, umfasst folgendes:

Erzeugen eines ersten und/oder eines zweiten drallbehafteten Gasstroms, der durch eine Gasaustrittsanordnung aus der Düsenanordnung in eine Sprührichtung austritt/austreten.

Erzeugen eines ersten Flüssigkeitsstroms, der durch einen Flüssigkeitsauslass in den ersten Gasstrom austritt und

Erzeugen eines zweiten Flüssigkeitsstroms, der durch einen zweiten Flüssigkeitsauslass in den ersten Gasstrom oder, falls vorhanden, in einen zweiten drallbehafteten Gasstrom austritt,

wobei der erste drallbehaftete Gasstrom und/oder der zweite drallbehaftete Gasstrom den ersten Flüssigkeitsstrom und den zweiten Flüssigkeitsstrom, vorzugsweise vollständig, umhüllen.

Insbesondere wird beim Beenden des Sprühvorgangs der erste Flüssigkeitsstrom und der zweite Flüssigkeitsstrom vor dem ersten Gasstrom und, falls vorhanden, dem zweiten Gasstrom unterbrochen. Vorzugsweise wird beim Beginnen des Sprühvorgangs der erste Gasstrom und, falls vorhanden, der zweite Gasstrom vor dem ersten Flüssigkeitsstrom und dem zweiten Flüssigkeitsstrom eingeleitet.

Sämtliche Merkmale und Vorteile, die in Bezug auf das erfindungsgemäße Instrument beschrieben wurden, sind ebenso auf das erfindungsgemäße Verfahren anwendbar.

Das erfindungsgemäße System weist ein Instrument der obigen Art und ein Versorgungsgerät zum Speisen des Instruments mit den benötigten Betriebsmitteln auf. Betriebsmittel können beispielsweise Fluide, wie Gase oder Flüssigkeiten sein, und/oder eine elektrische Spannung, insbesondere eine hochfrequente Wechselspannung.

Weitere Einzelheiten vorteilhafter Weiterbildungen oder Details der Erfindung ergeben sich aus den Zeichnungen, der Beschreibung sowie den Unteransprüchen. Es zeigen:
Figur 1 eine schematische Ansicht eines Ausführungsbeispiels des Instruments mit einem Versorgungsgerät;
Figur 2 eine Querschnittsansicht der Düsenanordnung des Instruments gemäß einem ersten Ausführungsbeispiel;
Figur 3 eine Querschnittsansicht der Düsenanordnung des Instruments gemäß einem zweiten Ausführungsbeispiel;
Figur 4 einen Längsschnitt der Düsenanordnung des Instruments gemäß dem ersten Ausführungsbeispiel;
Figur 5 einen Längsschnitt der Düsenanordnung des Instruments gemäß dem zweiten Ausführungsbeispiel;
Figur 6 eine distale Draufsicht auf die Düsenanordnung des Instruments gemäß einem dritten Ausführungsbeispiel;
Figur 7 einen Längsschnitt der Düsenanordnung des Instruments gemäß einem dritten Ausführungsbeispiel;
Figur 8 einen Längsschnitt der abgewandelten Düsenanordnung des Instruments gemäß dem dritten Ausführungsbeispiel; und
Figur 9 einen Steuerplan für einen Sprühvorgang.

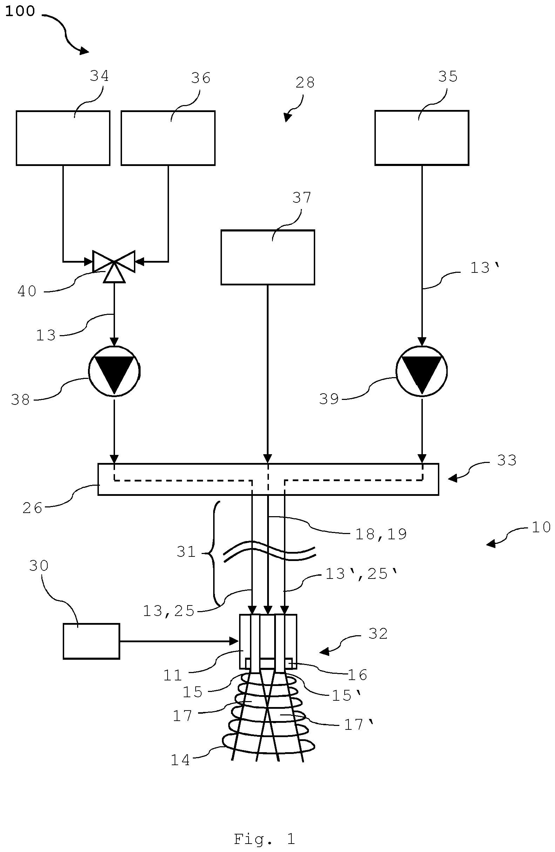

In Figur 1 ist ein System 100 zum Zuführen, Mischen und Versprühen von mehreren flüssigen Komponenten abgebildet. Das System 100 weist ein Instrument 10 und ein Versorgungsgerät 28 auf. Das Instrument 10 ist vorzugsweise zur minimalinvasiven Anwendung, insbesondere zur laparoskopischen Anwendung, ausgestaltet. Das Instrument 10 weist eine Düsenanordnung 11 auf, die distal am Instrument 10 angeordnet ist. Die Düsenanordnung 11 ist dazu eingerichtet, Flüssigkeiten 13 und 13' mittels eines ersten und/oder eines zweiten drallbehafteten Gasstroms 14, 14' zu versprühen.

Die Düsenanordnung 11 weist einen ersten Flüssigkeitsauslass 15 und einen zweiten Flüssigkeitsauslass 15' auf, durch die ein erster Flüssigkeitsstrom 17 der ersten Flüssigkeit 13, und ein zweiter Flüssigkeitsstrom 17' der zweiten Flüssigkeit 13' strömen, auf. Die Düsenanordnung 11 weist außerdem eine Gasaustrittsanordnung 16 auf, die dazu eingerichtet ist, den ersten und/oder zweiten Gasstrom 14, 14' auszugeben.

Das Instrument 10 weist einen länglichen Schaft 31 auf, in welchen die zu versprühenden Flüssigkeiten 13 und 13' sowie das Gas zum Erzeugen des drallbehafteten Gasstroms transportiert werden. Am proximalen Ende 33 des Instruments 10 weist das Instrument 10 eine Schnittstelleneinrichtung 26 auf. Die Schnittstelleneinrichtung 26 ist dazu eingerichtet, mit einem Versorgungsgerät 28 verbunden zu werden. Das Versorgungsgerät 28 stellt die Flüssigkeit 13 und die zweite Flüssigkeit 13' bereit.

Der längliche Schaft 31 des Instruments 10 weist eine Flüssigkeitskapillare 25, die der Flüssigkeit 13 zugeordnet ist, und eine zweite separate Flüssigkeitskapillare 25` auf, die der zweiten Flüssigkeit 13' zugeordnet ist. Über die Flüssigkeitskapillare 25 und 25' werden die Flüssigkeiten 13 und 13` von der Schnittstelleneinrichtung 26 am proximalen Ende 33 des Instruments 10 zu der Düsenanordnung 11 am distalen Ende 32 des Instruments 10 transportiert. In dem länglichen Schaft 31 des Instruments 10 ist außerdem ein Gaszuführkanal 18 angeordnet, der einen Gaszuführstrom 19 von der Schnittstelleneinrichtung 26 am proximalen Ende 33 zu der Düsenanordnung 11 am distalen Ende 32 des Instruments 10 transportiert.

Das Versorgungsgerät 28 weist eine erste Quelle 34 für eine erste Komponente, eine zweite Quelle 35 für eine zweite Komponente sowie eine dritte Quelle 36 für ein Verdünnungsmittel und eine vierte Quelle 37 für ein Gas auf. Bei der ersten Komponente kann es sich beispielsweise um ein Präpolymer handeln. Das Präpolymer in der ersten Quelle 34 kann eine relativ hohe Viskosität aufweisen, beispielsweise größer als 9 mPa·s, weshalb diesem in der Mischeinrichtung 40 Verdünnungsmittel aus der dritten Quelle 36 zugemischt werden kann. Bei dem Verdünnungsmittel kann es sich beispielsweise um Wasser handeln. Die mit dem Verdünnungsmittel vermischte erste Komponente bildet hierbei die erste Flüssigkeit 13, welche nach dem Vormischen über eine erste Dosiereinheit 38 an die Schnittstelleneinrichtung 26 ausgegeben werden kann. Bei der zweiten Komponente kann es sich um einen Katalysator handeln. Die zweite Komponente bildet die zweite Flüssigkeit 13', welche über eine separate Dosiereinheit 39 von dem Versorgungsgerät 28 an die Schnittstelleneinrichtung 26 ausgegeben werden kann. Bei der zweiten Flüssigkeit kann es sich beispielsweise um einen Katalysator für die erste Flüssigkeit handeln. Das Versorgungsgerät 28 weist außerdem eine vierte Quelle 37 für das Gas auf. Bei dem Gas handelt es sich vorzugsweise um Kohlenstoffdioxid oder Luft, oder ein Inertgas, z.B. Stickstoff oder Edelgase, wie beispielsweise Argon oder dergleichen. Das Gas aus der vierten Quelle 37 wird von dem Versorgungsgerät 28 über die Schnittstelleneinrichtung 26 für das Instrument 10 bereitgestellt. Bei den Quellen 34, 35, 36, 37 kann es sich beispielsweise um Reservoire oder Tanks handeln, in welchen die Komponenten gelagert werden. Alternativ kann es sich bei den Quellen 34, 35, 36, 37 auch um Anschlüsse an eine Versorgungsleitung der Komponente handeln.

Das Instrument 10 weist außerdem eine Steuereinheit 30 auf, die dazu eingerichtet ist, die Flüssigkeitsströme 17, 17' bei einem Beenden des Sprühvorgangs vor den Gasströmen 14, 14', 14" zu unterbrechen. Ebenso ist die Steuereinheit 30 dazu eingerichtet, die Flüssigkeitsströme 17 und 17' beim Beginn des Sprühvorgangs nach den Gasströmen 14, 14', 14" zu aktivieren.

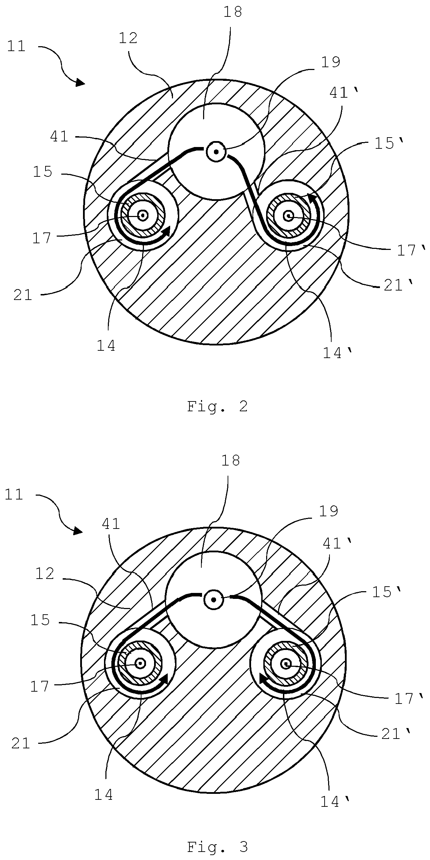

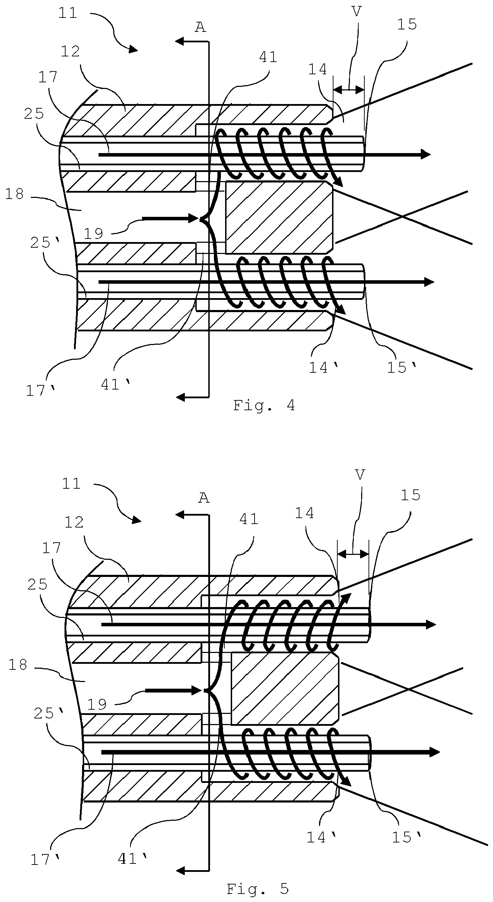

Figur 2 zeigt einen Querschnitt durch den Düsenkörper 12 einer Düsenanordnung 11 des Instruments 10 gemäß einem ersten Ausführungsbeispiel entlang der in Figur 4 eingezeichneten Schnittebene A. In dem Düsenkörper 12 ist ein Gaszuführkanal 18 vorgesehen, durch den der Gaszuführstrom 19 geführt wird. Der Gaszuführstrom 19 zeigt aus der Zeichenebene heraus.

In dem Düsenkörper 12 ist außerdem ein erster Flüssigkeitsauslass 15 und ein zweiter Flüssigkeitsauslass 15' angeordnet. Durch den ersten Flüssigkeitsauslass 15 wird der erste Flüssigkeitsstrom 17 ausgeleitet. Der zweite Flüssigkeitsauslass 15' ist wiederum für den zweiten Flüssigkeitsstrom 17' vorgesehen. Die Flüssigkeitsauslässe 15 und 15' sind jeweils von einer Gasaustrittsöffnung 21 und 21` umgeben, die zusammen die Gasaustrittsanordnung 16 bilden.

Zudem ist in dem Düsenkörper 12 ein Drallerzeugungsmittel untergebracht. Das Drallerzeugungsmittel wird in diesem Ausführungsbeispiel durch Verbindungskanäle 41, 41` gebildet, über die die Gasaustrittsöffnungen 21 und 21' mit dem Gaszuführkanal 18 fluidisch verbunden sind. Die Verbindungskanäle 41 sind derart angeordnet, dass sich der zentrale Gaszuführstrom 19 in zwei Teilgasströme aufteilt. Die Teilgasströme werden von den Verbindungskanälen 41, 41' derart geführt in die Gasaustrittsöffnungen 21, 21` eingeleitet, dass sich in der ersten Gasaustrittsöffnung 21 ein erster drallbehafteter Gasstrom 14 und in der zweiten Gasaustrittsöffnung 21` ein zweiter drallbehafteter Gasstrom 14' ausbildet. Die Teilgasströme werden durch die Verbindungskanäle 41, 41' vorzugsweise tangential zu den Flüssigkeitsauslässen 15, 15' in die Gasaustrittsöffnungen 21, 21` der Gasaustrittsanordnung 16 eingeleitet. Die Teilströme können in einem Einströmwinkel relativ zu einer Querschnittsebene des Düsenkörpers 12 in die Gasaustrittsöffnungen 21, 21` einströmen. Die Verbindungskanäle 41, 41' sind in diesem Ausführungsbeispiel derart angeordnet, dass der erste und der zweite Gasstrom 14, 14' gleichsinnig rotierend strömen. Zusätzlich oder alternativ können in den Gasaustrittsöffnungen 21, 21` Drallkörper 20, 20' angeordnet sein, die den ersten Gasstrom 14 und den zweiten Gasstrom 14' mit einen Drall versehen.

In Figur 3 ist ein Querschnitt entlang der Schnittebene A aus Figur 5 gemäß einem zweiten Ausführungsbeispiel abgebildet. Für das in Figur 3 gezeigte Beispiel gilt das zuvor beschriebene unter Bezugnahme der Bezugszeichen endsprechend, wobei jedoch die Verbindungskanäle 41 derart angeordnet sind, dass die Gasströme 14 und 14' entgegengesetzt rotieren. Figur 3 zeigt einen Querschnitt entlang der Schnittebene A aus Figur 5.

In Figur 4 ist ein Längsschnitt der Düsenanordnung 11 gemäß dem ersten Ausführungsbeispiel veranschaulicht. In dem Düsenkörper 12 ist der Gaszuführkanal 18 untergebracht, durch den der Gaszuführstrom 19 der Düsenanordnung 11 zugeführt wird. In dem Düsenkörper 12 sind außerdem die erste Flüssigkeitskapillare 25 und die zweite Flüssigkeitskapillare 25' angeordnet, durch die der erste Flüssigkeitsstrom 17 und der zweite Flüssigkeitsstrom 17` geführt werden. Die erste Flüssigkeitskapillare 25 endet in dem ersten Flüssigkeitsauslass 15 und die zweite Flüssigkeitskapillare 25' endet in dem zweiten Flüssigkeitsauslass 15'.

In einem Endabschnitt der Düsenanordnung 11 ist eine Gasaustrittsöffnung 21 konzentrisch um den ersten Flüssigkeitsauslass 15 angeordnet. Die erste Gasaustrittsöffnung 21 ist in diesem Beispiel als ein Ringspalt ausgestaltet. Der zentrale Gaszuführkanal 18 ist über einen Verbindungskanal 41 mit der ersten Gasaustrittsöffnung 21 verbunden. Im Endabschnitt der Düsenanordnung 11 ist auch eine zweite Gasaustrittsöffnung 21` um den zweiten Flüssigkeitsauslass 15' konzentrisch angeordnet. Auch die zweite Gasaustrittsöffnung 21` ist ebenfalls als Ringspalt ausgestaltet, wobei die zweite Gasaustrittsöffnung 21 über einen Verbindungskanal 41' mit dem Gaszuführkanal 18 verbunden ist. Die Verbindungskanäle 41 sind derart angeordnet, dass der Gaszuführstrom 19 in zwei Teilströme unterteilt wird, denen ein Drall aufgeprägt wird, der entgegen dem Uhrzeigersinn zeigt. Der erste drallbehaftete Gasstrom 14 und der zweite drallbehaftete Gasstrom 14` rotieren gleichsinnig. Der erste Flüssigkeitsauslass 15 und der zweite Flüssigkeitsauslass 15' stehen um den Abstand V distal von dem Instrument 10 hervor.

In Figur 5 ist ein Längsschnitt des zweiten Beispiels für die Düsenanordnung 11 gezeigt. Auch in Bezug auf Figur 5 gilt das bisher beschriebene unter Zugrundelegung der Bezugszeichen entsprechend. Figur 5 unterscheidet sich von Figur 4 dadurch, dass die Verbindungskanäle 41 derart angeordnet sind, dass der drallbehaftete Gasstrom 14 und der zweite drallbehaftete Gasstrom 14' gegensinnig rotieren.

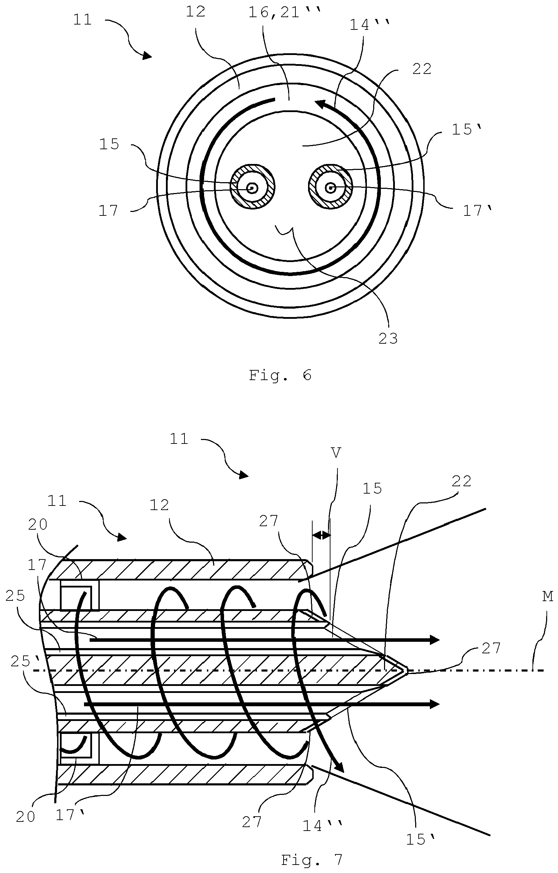

Figur 6 zeigt eine distale Draufsicht der Düsenanordnung 11 gemäß dem dritten Ausführungsbeispiel. In diesem Ausführungsbeispiel sind die Flüssigkeitsauslässe 15 und 15' in einem gemeinsamen Strömungskörper 22 angeordnet. Der Strömungskörper 22 wird von der Gasaustrittsanordnung 16 umschlossen. Die Gasaustrittsanordnung 16 weist hierfür eine den Strömungskörper 22 umschließende Gasaustrittsöffnung 21" auf. Diese Gasaustrittsöffnung 21" ist ebenfalls als Ringspalt ausgebildet. Zur Erzeugung des drallbehafteten den Strömungskörper 22 umgebenden Gasstroms 14" ist in dem Düsenkörper 12 ein Drallkörper 20 vorgesehen, der dem Gaszuführstrom 19 einen zusätzlichen Drehimpuls aufprägt.

Figur 7 zeigt ein Ausführungsbeispiel für die Düsenanordnung, bei der die beiden Flüssigkeitsauslässe 15 und 15' in dem Strömungskörper 22 angeordnet sind. Der Strömungskörper 22 wird von der Gasaustrittsöffnung 21" umhüllt. In der Gasaustrittsöffnung 21" ist mindestens ein Drallkörper 20 angeordnet, der dem Gaszuführstrom 19 einen Drehimpuls aufprägt. Die Außenkontur des Strömungskörpers 22 ist als Kegel ausgebildet. Es ist ebenfalls möglich, dass die Außenkontur des Strömungskörpers 22 als Kegelstumpf ausgebildet ist. Die Oberfläche 23 des Strömungskörpers 22 weist eine zusätzliche Beschichtung 27 auf. Die Beschichtung kann beispielsweise hydrophobe Eigenschaften aufweisen.

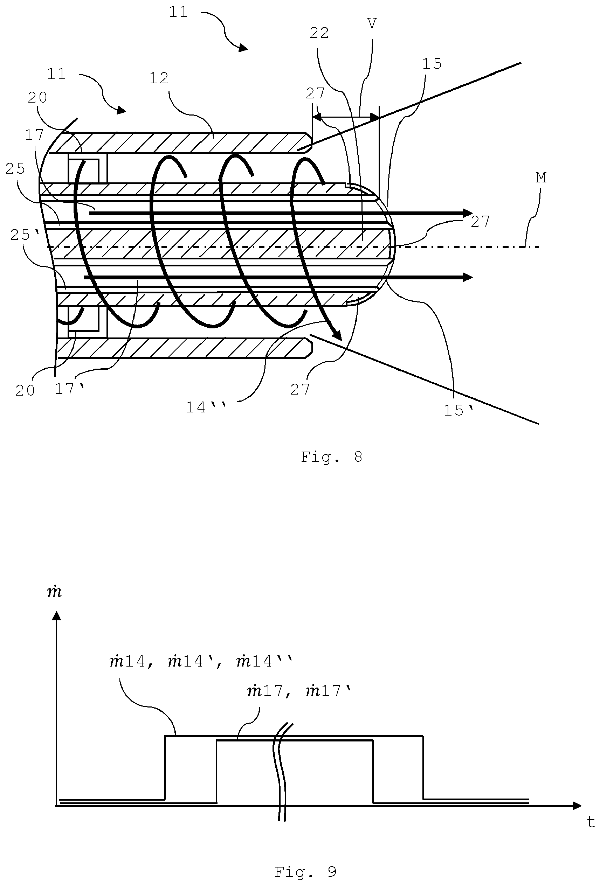

Figur 8 zeigt ein zweites Beispiel für eine Düsenanordnung, bei der die Flüssigkeitsauslässe 15 und 15' in einem Strömungskörper 22 vorgesehen sind. Der Strömungskörper 22 weist eine konvexe Außenkontur auf. Auch in dem in Figur 8 gezeigten Beispiel ist eine Beschichtung der Oberfläche 23 des Strömungskörpers 22 vorgesehen, die beispielsweise hydrophob ausgestaltet sein kann.

In Figur 9 sind die Massenströme *ṁ* des ersten Gasstroms 14 bzw. zweiten Gasstroms 14` bzw. dritten Gasstroms 14" und dem ersten Flüssigkeitsstrom 17 bzw. zweiten Flüssigkeitsstrom 17' über der Zeit t dargestellt. Die jeweiligen Massenströme werden mit *ṁ*14, *ṁ*14', *ṁ*14" und *ṁ*17, *ṁ*17' bezeichnet. Beim Beginn des Sprühvorgangs wird zuerst der Massenstrom *ṁ*14, *ṁ*14', *ṁ*14" des Gasstromes 14 bzw. 14' bzw. 14" eingeleitet, bevor die Massenströme *ṁ*17, *ṁ*17' der Flüssigkeitsströme 17, 17' eingeleitet werden. Beim Beenden des Sprühvorgangs werden entsprechend die Massenströme *ṁ*17, *ṁ*17' der Flüssigkeitsströme 17, 17' vor den Massenströmen *ṁ*14, *ṁ*14', *ṁ*14" der Gasströme 14, 14', 14" unterbrochen.

Das erfindungsgemäße Instrument 10 dient dazu, mehrere miteinander reaktive Flüssigkeiten 13, 13` mittels mindestens eines drallbehafteten Gasstroms 14, 14', 14" zu Versprühen und außerhalb der Düsenanordnung 11 des Instruments 10 zu vermischen. Die Düsenanordnung 11 des Instruments weist einen Düsenkörper 12 mit mindestens einem Flüssigkeitsauslass 15, 15', durch den eine oder mehrere Flüssigkeiten 13, 13` ausgebbar sind, und einer Gasaustrittsanordnung 16 auf, durch die der mindestens eine drallbehaftete Gasstrom 14, 14', 14" ausgebbar ist, wobei die Gasaustrittsanordnung den mindestens einen Flüssigkeitsauslass, vorzugsweise vollständig, umschließt.

### Bezugszeichenliste:

- 10: Instrument
- 11: Düsenanordnung
- 12: Düsenkörper
- 13: erste Flüssigkeit (Präpolymer)
- 13': zweite Flüssigkeit (Katalysator)
- 14: erster drallbehafteter Gasstrom
- 14`: zweiter drallbehafteter Gasstrom
- 14": drallbehafteter Gasstrom
- 15: erster Flüssigkeitsauslass
- 15': zweiter Flüssigkeitsauslass
- 16: Gasaustrittsanordnung
- 17: erster Flüssigkeitsstrom
- 17': zweiter Flüssigkeitsstrom
- 18: Gaszuführkanal
- 19: Gaszuführstrom
- 20: Drallkörper
- 21: erste Gasaustrittsöffnung
- 21': zweite Gasaustrittsöffnung
- 21": Gasaustrittsöffnung
- 22: Strömungskörper
- 23: Oberfläche des Strömungskörper
- 24: Schaft des Instruments
- 25: erste Flüssigkeitskapillar
- 25`: zweite Flüssigkeitskapillar
- 26: Schnittstelleneinrichtung
- 27: Beschichtung
- 28: Versorgungsgerät
- 29: erster Flüssigkeitszuführstrom
- 29': zweiter Flüssigkeitszuführstrom
- 30: Steuereinheit
- 31: länglicher Schaft des Instruments
- 32: distales Ende des Instruments
- 33: proximales Ende des Instruments
- 34: erste Quelle für die erste Komponente
- 35: zweite Quelle für die zweite Komponente
- 36: dritte Quelle für das Verdünnungsmittel (Wasser)
- 37: vierte Quelle für das Gas
- 38: erste Dosiereinheit (Pumpe)
- 39: zweite Dosiereinheit (Pumpe)
- 40: Mischeinrichtung
- 41: Verbindungskanäle
- A: Schnittebene
- *ṁ*: Massenströme
- t: Zeit
- V: Abstand

## Patentansprüche

1. Instrument (10), vorzugsweise zur minimalinvasiven Anwendung, mit einer distal am Instrument (10) angeordneten Düsenanordnung (11), die dazu eingerichtet ist, Flüssigkeiten (13, 13') mittels eines drallbehafteten Gasstroms (14, 14', 14") zu versprühen,
wobei die Düsenanordnung (11) einen Düsenkörper (12) mit einem Flüssigkeitsauslass (15, 15') und einer Gasaustrittsanordnung (16) aufweist,
wobei das Instrument (10) dazu eingerichtet ist, einen Flüssigkeitsstrom (17, 17') durch den Flüssigkeitsauslass (15, 15') und den drallbehafteten Gasstrom (14, 14', 14") durch die Gasaustrittsanordnung (16) auszugeben, wobei die Gasaustrittsanordnung (16) den Flüssigkeitsauslass (15, 15') umschließt.

2. Instrument (10) nach Anspruch 1, **dadurch gekennzeichnet, dass** die Düsenanordnung (11) den ersten und einen zweiten Flüssigkeitsauslass (15, 15') aufweist, durch welchen ein zweiter Flüssigkeitsstrom (17') ausgebbar ist, wobei der zweite Flüssigkeitsauslass (15') von dem Flüssigkeitsauslass (15) beabstandet angeordnet ist.

3. Instrument (10) nach Anspruch 2, **dadurch gekennzeichnet, dass** die Gasaustrittsanordnung (16) eine erste Gasaustrittsöffnung (21) aufweist, welche konzentrisch zu dem ersten Flüssigkeitsauslass (15) angeordnet ist.

4. Instrument (10) nach Anspruch 3, **dadurch gekennzeichnet, dass** die Gasaustrittsanordnung (16) eine zweite Gasaustrittsöffnung (21`) aufweist, welche konzentrisch zu dem zweiten Flüssigkeitsauslass (15') angeordnet ist, wobei die erste Gasaustrittsöffnung (21) und die zweite Gasaustrittsöffnung (21`) jeweils dazu eingerichtet sind, einen drallbehaftete Gasstrom (14, 14') auszugeben.

5. Instrument (10) nach Anspruch 4, **dadurch gekennzeichnet, dass** die durch die erste Gasaustrittsöffnung (21) und die durch die zweite Gasaustrittsöffnung (21`) austretenden, drallbehafteten Gasströme (14, 14') mit einem gleichsinnig rotierenden oder einem gegeneinander rotierenden Drall beaufschlagt sind.

6. Instrument (10) nach einem der Ansprüche 2 bis 5, **dadurch gekennzeichnet, dass** der erste Flüssigkeitsauslass (15) und der zweite Flüssigkeitsauslass (15') in einem gemeinsamen Strömungskörper (22) voneinander beabstandet angeordnet sind, welcher eine Mittelachse (M) definiert.

7. Instrument (10) nach Anspruch 6, **dadurch gekennzeichnet, dass** die Gasaustrittsanordnung (16) eine den Strömungskörper (22) umschließende Gasaustrittsöffnung (21") aufweist, die vorzugsweise konzentrisch zur Mittelachse (M) des Strömungskörpers (22) angeordnet ist.

8. Instrument (10) nach Anspruch 6 oder 7, **dadurch gekennzeichnet, dass** der Strömungskörper (22) eine konvex oder kegelförmig ausgestaltete Außenkontur aufweist.

9. Instrument (10) nach einem der Ansprüche 2 bis 8, **dadurch gekennzeichnet, dass** der erste, der zweite und/oder der den Strömungskörper umgebende drallbehaftete Gasstrom (14, 14', 14") den ersten Flüssigkeitsstrom (17) und den zweiten Flüssigkeitsstrom (17'), vorzugsweise vollständig, umhüllt/en.

10. Instrument (10) nach einem der vorhergehenden Ansprüche, **dadurch gekennzeichnet, dass** das Instrument (10) einen Gaszuführkanal (18) aufweist, welcher in den Düsenkörper (12) mündet und über welchen die Gasaustrittsanordnung (16) mit einen Gaszuführstrom (19) versorgbar ist, wobei in dem Düsenkörper (12) mindestens ein Drallerzeugungsmittel, vorzugsweise ein Drallkörper (20), untergebracht ist, der dazu eingerichtet ist, den Gaszuführstrom (18) mit einem vorbestimmten Drall zu beaufschlagen.

11. Instrument (10) nach einem der vorhergehenden Ansprüche, **dadurch gekennzeichnet, dass** der Flüssigkeitsauslass (15) und/oder der zweite Flüssigkeitsauslass (15') distal von dem Instrument (10) vorstehend angeordnet ist/sind.

12. Instrument (10) nach einem der vorhergehenden Ansprüche, **dadurch gekennzeichnet, dass** das Instrument (10) ferner einen länglichen Schaft (24) aufweist, in welchem der Gaszuführkanal (18) und Flüssigkeitskapillare (25, 25') für jeden Flüssigkeitsauslass (15, 15') separat zueinander verlaufen, wobei das Instrument (10) außerdem eine proximal angeordnete Schnittstelleneinrichtung (26) aufweist, die mit einem Versorgungsgerät (28) verbindbar ist, welches dazu eingerichtet ist, das Instrument (10) mit den Flüssigkeiten (13, 13') sowie dem Gaszuführstrom (18) zu versorgen.

13. Instrument (10) nach einem der vorhergehenden Ansprüche, **dadurch gekennzeichnet, dass** das Instrument (10) eine Steuereinheit (30) aufweist, die dazu eingerichtet ist, den Flüssigkeitsstrom (17) sowie den zweiten Flüssigkeitsstrom (17') bei einem Beenden des Sprühvorgangs vor dem Gasstrom (14) und dem zweiten Gasstrom (14') zu unterbrechen.

14. Verfahren zum Versprühen von Flüssigkeiten (13, 13') mittels eines Instruments (10), vorzugsweise nach einem der vorhergehenden Ansprüche, umfassend:
Erzeugen eines drallbehafteten Gasstroms (14, 14', 14"), der durch eine Gasaustrittsanordnung (16) aus der Düsenanordnung (11) in eine Sprührichtung austritt,
Erzeugen eines ersten Flüssigkeitsstroms (17), der durch einen Flüssigkeitsauslass (15) austritt, und
Erzeugen eines zweiten Flüssigkeitsstroms (17`), der durch einen Flüssigkeitsauslass (15') austritt,
wobei der drallbehaftete Gasstrom (14, 14', 14") den mindestens einen Flüssigkeitsstrom (17) umhüllt.

15. System (100) mit einem Instrument (10) nach einem der Ansprüche 1 bis 13 sowie einem Versorgungsgerät (28) zum Speisen des Instruments (10) mit den benötigten Betriebsmitteln.
